# EUROPÄISCHE PATENTSCHRIFT

(11) **EP 2 004 278 B1**
(45) Veröffentlichungstag und Bekanntmachung des Hinweises auf die Patenterteilung: **05.08.2015**
(21) Anmeldenummer: 07703069.0
(22) Anmeldetag: 26.01.2007
(51) Int. Cl.: A61N 1/05, B81B 3/00

(54) **VERFAHREN ZUR HERSTELLUNG VON IMPLANTATSTRUKTUREN ZUR KONTAKTIERUNG ODER ELEKTROSTIMULATION VON LEBENDEN GEWEBEZELLEN ODER NERVEN**
METHOD FOR PRODUCING IMPLANT STRUCTURES FOR CONTACTING OR ELECTRICALLY STIMULATING LIVING TISSUE CELLS OR NERVES
PROCÉDÉ DE FABRICATION DE STRUCTURES D'IMPLANT POUR LA MISE EN CONTACT OU L'ÉLECTROSTIMULATION DE CELLULES TISSULAIRES VIVANTES OU DE NERFS

(30) Priorität: 13.04.2006 DE 102006017549
(43) Veröffentlichungstag der Anmeldung: 24.12.2008
(73) Patentinhaber: Pixium Vision SA, 75012 Paris (FR)
(72) Erfinder: TIEDTKE, Hans-Jürgen, 53227 Bonn (DE)
(74) Vertreter: Graf von Stosch, Andreas
(86) Internationale Anmeldenummer: PCT/EP2007/000686
(87) Internationale Veröffentlichungsnummer: WO 2007/118527

(56) Entgegenhaltungen:
- EP-A- 1 096 026
- EP-A- 1 557 396
- WO-A-01/04964
- WO-A-2005/065432
- US-A- 5 720 099
- US-A1- 2005 239 272
- US-A1- 2006 053 625
- HOLMES A S ET AL: "Low cost LIGA processes" IEE MICROENGINEERING TECHNOLOGIES AND HOW TO EXPLOIT THEM, 8. April 1977 (1977-04-08), Seiten 2/1-4, XP006509642 BIRMINGHAM, UK
- BANKS D: "General introduction to micromachining" IEE MICROENGINEERING TECHN OLOGIES AND HOW TO EXPLOIT THEM (REF. 1997/076) 8 APRIL 1997 BIRMINGHAM, UK, 8. April 1997 (1997-04-08), Seiten 1/1-6, XP006509641 London, UK

## Beschreibung

Die vorliegende Erfindung bezieht sich allgemein auf Implantatstrukturen und Systeme, die der Kontaktierung von lebendem Gewebe oder Nerven dienen. Die Erfindung betrifft insbesondere ein Verfahren zur Herstellung von mehrlagigen Implantatstrukturen (Mehrschichtsystem-Implantatstrukturen bzw. Multileyer) mit mindestens einer Leiterbahnebene mit elektrischen Leiterbahnen zur elektrischen Kontaktierung von Implantaten, die der Kontaktierung oder Elektrostimulation, von lebenden Gewebezellen oder Nerven dienen.

Es sind Vorrichtungen in Form von Implantaten zur Stimulation von lebendem Gewebe bekannt, wie z.B. Implantate für die Netzhaut (Retina) des menschlichen Auges oder für das menschliche Innenohr. Solche Implantate umfassen in der Regel eine Anzahl von Stimulationselektroden, über die elektrische Stimulationsimpulse an das umgebende Gewebe bzw. die Zellen abgegeben werden, um so die Nerven zu stimulieren und damit deren Funktion wiederherzustellen bzw. zu verbessern.

Bekannte Implantate sind häufig Bestandteil von Systemen, die elektrische oder elektronische Komponenten zu sensorischen oder diagnostischen Zwecken umfassen, wie z.B. die elektrische Messung von Körperfunktionen, Blutdruck, Blutzucker oder der Temperatur. Stimulations-Systeme können Komponenten zu aktorischen Zwecken enthalten, wie z.B. zur Elektrostimulation, Defibrillation, Schallaussendung oder Ultraschallaussendung. Solche Systeme umfassen in der Regel ein Substrat in Form einer Leiterplatte, auf der die elektronischen Bauelemente angeordnet sind, mit elektrischen Kontakten, die im direkten oder indirekten Kontakt zum Körpergewebe stehen, wie z.B. Nervengewebe und Muskelgewebe oder zu Körperflüssigkeiten.

Um die Abmessungen der elektrischen oder elektronischen Komponenten möglichst klein zu halten, werden neben keramischen Substraten zunehmend auch flexible Leiterplatten aus Kunststoffen eingesetzt. Solche flexiblen Leiterplatten lassen sich mit Hilfe von etablierten Prozessen zur Herstellung von Mikrochips in sehr feinen Abmessungen mit einer Schichtdicke der Leiterbahnen bis zu einigen Hundert Nanometer und einer Leiterbahnbreite von beispielsweise wenigen Mikrometer strukturieren.

Eine solche flexible Leiterplatte besteht in der Regel aus einer oder mehreren isolierenden Schichten beispielsweise aus Polyimid, Parylene, anderen Kunststoffen oder Isolatoren, auf denen Leiterbahnen, Kontaktflächen oder gegebenenfalls Durchkontaktierungen zwischen mehreren Leiterbahnebenen eingerichtet sind. Zur elektrischen Kontaktierung der Leiterbahnen sind entsprechende Kontaktstellen vorgesehen, über die beispielsweise externe elektrische Leitungen und/oder externe Bauelemente angeschlossen werden können, um die elektronischen Komponenten auf der Leiterplatte mit externen Komponenten des Stimulations-Systems zu verbinden.

Zur Herstellung der flexiblen Leiterplatten werden in der Regel Mehrschichtsysteme bzw. sogenannte Multilayer erzeugt, die aus mehreren Schichten bestehen. Diese Schichten des Multilayers können eine Anzahl von Leiterbahnebenen bzw. Leiterbahnschichten umfassen, in denen elektrisch leitende Leiterbahnen vorgesehen sind, sowie eine Anzahl von Isolationsschichten aufweisen, die beispielsweise zwischen zwei Leiterbahnschichten angeordnet sind, um diese elektrisch voneinander zu isolieren.

Solche Multilayer enthalten häufig eine größere Anzahl von Einzelstrukturen, die auf einem gemeinsamen Substrat gleichzeitig prozessiert und aufgebaut werden. Nach Abschluss des Herstellungsverfahrens muss der Multilayer zur weiteren Bearbeitung von dem Substrat abgelöst werden. In der Literatur werden verschiedene Verfahren zum Ablösen der prozessierten Mehrschichtsysteme von dem Substrat beschrieben, bei denen eine Ablöseschicht aufgebracht und nach Fertigstellung des Herstellungsprozesses mit einem geeigneten Mittel entfernt wird. Die Ablöseschicht kann z.B. aus Photolack, SiO₂, Polymeren, Metallen etc. bestehen.

Solche Verfahren werden beispielsweise beschrieben in US 2006/0053625 oder US 5 720 099.

Es ist jedoch kein Verfahren bekannt, bei dem die Einzelstrukturen beim Ablösen vom Substrat im Verbund erhalten bleiben. Bei den bekannten Herstellungs- und Ablöseverfahren lassen sich die Einzelstrukturen nur vereinzelt ablösen. Die Weiterverarbeitung der vereinzelten Strukturen ist jedoch mit einem höheren Aufwand und damit auch höheren Kosten verbunden als wenn die Multilayer auch nach ihrer Herstellung noch im Verbund vorliegen würden.

Es ist deshalb eine Aufgabe der vorliegenden Erfindung, ein Verfahren zur Herstellung von Multilayern zu schaffen, das ein leichtes Ablösen der erzeugten Einzelstrukturen vom Substrat ermöglicht. Eine weitere Aufgabe der vorliegenden Erfindung besteht darin, ein Verfahren zu schaffen, bei dem die auf einem Substrat erzeugten Einzelstrukturen gemeinsam vom Substrat gelöst werden können und dabei im Verbund erhalten bleiben.

Diese Aufgabe wird durch das erfindungsgemäße Verfahren mit den Merkmalen gemäß Anspruch 1 gelöst. Vorteilhafte Weiterbildungen der Erfindung sind jeweils in den Unteransprüchen angegeben.

Nach der vorliegenden Erfindung wird die oben genannte Aufgabe gelöst durch ein Verfahren zum Herstellen von mehrlagigen Implantatstrukturen mit mindestens einer Leiterbahnebene mit elektrischen Leiterbahnen zur elektrischen Kontaktierung von bspw. Implantaten, die bspw. der Kontaktierung oder

Elektrostimulation von lebenden Gewebezellen oder Nerven dienen, umfassend die folgenden Schritte:
- Erzeugen einer ersten Metallschicht auf einem Substrat,
- Erzeugen einer zweiten Metallschicht über der ersten Metallschicht,
- Herstellen eines Multilayers umfassend eine Anzahl von mehrlagigen Implantatstrukturen über der zweiten Metallschicht,
- Entfernen der ersten Metallschicht zwischen dem Substrat und der zweiten Metallschicht und
- Ablösen der mehrlagigen Implantatstrukturen in einem zusammenhängenden Verbund von dem Substrat.

Auf diese Weise wird mit dem erfindungsgemäßen Verfahren zwischen dem Multilayer und dem Substrat eine Ablöseschicht aus zwei Metallschichten erzeugt, die beim Ablösen des fertig prozessierten Multilayers durch einen nasschemischen Unterätzungsprozess als Opferschicht dienen. Dadurch wird eine gleichmäßige und zuverlässige Trennung der fertigen Multilayer vom Substrat erzielt. Das erfindungsgemäße Verfahren hat damit den Vorteil, dass alle auf dem Substrat erzeugten Einzel-Implantatstrukturen gemeinsam von dem Substrat im Verbund und nicht einzeln abgelöst werden. Dies erleichtert die nachfolgende Aufbau- und Verbindungstechnik im Hinblick auf Automatisierung und Prozessierung.

Dieser vorteilhafte Effekt des erfindungsgemäße Verfahren ist dadurch begründet, dass die Ablösung der erzeugten Multilayer über eine Ablöseschicht aus Metall erfolgt, die aus einer ersten und einer zweiten Metallschicht besteht und zusammen durch einen oder mehrere geeignete Ätzprozesse entfernt werden, wenn der Multilayer fertig prozessiert ist. Dieser Ablösungsvorgang wird durch die Auswahl von geeigneten Metallschichten begünstigt und dadurch ein leichtes Ablösen der fertig prozessierten Multilayer vom Substrat ermöglicht. Das Substrat kann beispielsweise aus Silizium, Glas oder Keramik bestehen.

Während die erste Metallschicht (Opferschicht) vorzugsweise aus Chrom gefertigt ist, wird das Material, aus dem die zweite Metallschicht erzeugt wird, vorzugsweise so gewählt, dass das Material der zweiten Metallschicht aufgrund seiner Position in der Spannungsreihe der Elemente in Bezug auf das Material der ersten Metallschicht eine Potentialdifferenz zu der ersten Metallschicht bewirkt. Dadurch ergibt sich eine elektrochemische Spannung zwischen der ersten Metallschicht (Opferschicht) und der zweiten Metallschicht, welche die Auslösung der Opferschicht beim Unterätzungsprozess und damit die Ablösung der Implantatstrukturen vom Substrat begünstigt. Als Material für die zweite Metallschicht kann beispielsweise Gold, Silber, Palladium oder ein anderes Edelmetall verwendet werden.

Bei hohen Prozesstemperaturen kann es jedoch zwischen der ersten und zweiten Metallschicht zu Diffusionsprozessen kommen, die eine Ablösung der fertigen Multilayer vom Substrat durch Unterätzung der Opferschicht behindern. Denn bei hohen Temperaturen kann das Gold aus der zweiten Metallschicht in die erste Metallschicht diffundieren und/oder das Chrom aus der ersten Metallschicht in die zweite Metallschicht diffundieren. Dadurch bildet sich an der Grenze zwischen der ersten und zweiten Metallschicht eine Legierung, die durch einen Ätzvorgang nicht mehr aufzulösen ist. Besonders vorteilhaft ist es daher, wenn zwischen der ersten und zweiten Metallschicht eine dritte Metallschicht eingefügt wird, die somit als Diffusionssperre zwischen der ersten und zweiten Metallschicht dient.

Diese zwischen der ersten und zweiten Metallschicht angeordnete Diffusionssperre der dritten Metallschicht verhindert ein Diffundieren von Gold aus der zweiten Metallschicht in die erste Metallschicht. Aufgrund der Diffusionssperre lassen sich die Multilayer auch bei Temperaturen über 250°C prozessieren. Die dritte Metallschicht erfüllt ihre Funktion als Diffusionssperre zwischen der ersten und der zweiten Metallschicht besonders gut, wenn die dritte Metallschicht aus Titan oder Wolframtitan hergestellt ist. Das Einfügen der dritten Metallschicht beeinflusst das den Ablöseprozess der ersten Metallschicht nicht negativ.

Die Entfernung einer Metallschicht bei erfindungsgemäßen Verfahren erfolgt typischerweise über einen Ätzprozeß. Für einen Ätzprozeß wird typischerweise ein für das jeweilige Metall der zu ätzenden Metallschicht charakteristisches und insoweit selektives Ätzmittel verwendet, mit dessen Hilfe typischerweise jeweils nur eine Metallschicht (teil)entfernt werden kann. Im Falle der Ätzung der Opferschicht (Metallschicht 1) wird, sofern diese Schicht in der bevorzugten Ausführungsform aus Chrom besteht, insbesondere eine saure wässrige Lösung von Cerammoniumnitrat eingesetzt, wobei zur Ansäuerung typischerweise Essigsäure verwendet wird. Die Metallschicht 3, sofern bspw. aus Titan bestehend, wird typischerweise mit stark verdünnter Flusssäure geätzt, sofern aus Wolframtitan bestehend, hingegen typischerweise mit Wasserstoffperoxid. Dem Fachmann sind die für ein bestimmtes Metall jeweils selektiven Ätzmedien bekannt.

Bei einer bevorzugten Ausführungsform wird der Multilayer, der mehrere Implantatstrukturen umfasst, von einen starren Rahmen, beispielweise in Form eines festen Rings umgeben, der um die Implantatstrukturen des Multilayers herum, vorzugsweise an der Außenkante des Multilayers ausgebildet bzw. aufgebracht wird. Dabei sind die Einzel-Implantatstrukturen beim Ablösen von dem Substrat über dünne Stege miteinander verbunden und in dem steifen Ring aufgespannt, was eine einfache Handhabung der Einzel-Implantatstrukturen ermöglicht. Dies erleichtert auch die nachfolgende Aufbau- und Verbindungstechnik bei der Weiterverarbeitung der Multilayer im Hinblick auf Automatisierung und Prozessierung. Im Laufe der Weiterverarbeitung können dann die Stege getrennt werden, um die fertigt aufgebauten Einzelstrukturen zu erhalten.

Bei einer bevorzugten Ausführungsform des erfindungsgemäßen Verfahrens wird bei der Prozessierung des Multilayers mindestens eine elektrisch isolierende Materialschicht erzeugt, auf der eine Leiterbahn-Schicht mit mindestens einer Leiterbahn angeordnet ist.

Bei einer anderen bevorzugten Ausführungsform der vorliegenden Erfindung besteht die flexible Leiterplatte für ein Implantat aus mehreren Schichten eines isolierenden Materials mit dazwischen oder darunter oder darauf angeordneten Leiterbahnen.

Eine weitere Aufgabe der vorliegenden Erfindung besteht darin, ein Zwischenprodukt zur Herstellung von Implantatstrukturen bereitzustellen, das sich durch gute Handhabbarkeit und einfache Weiterbearbeitung auszeichnet. Diese Aufgabe wird gelöst durch ein Zwischenprodukt zur Herstellung von Implantatstrukturen, das aus den folgenden Schichten aufgebaut ist:
- einem Substrat,
- einer ersten Metallschicht auf dem Substrat,
- einer zweiten Metallschicht über der ersten Metallschicht und
- mindestens einem Multilayer über der zweiten Metallschicht, wobei der Multilayer eine Anzahl von zusammenhängenden mehrlagigen Implantatstrukturen mit elektrischen Leiterbahnen zur elektrischen Kontaktierung von Implantaten, die der Kontaktierung oder Elektrostimulation von lebenden Gewebezellen oder Nerven dienen, umfasst.

Eine solche Implantatstruktur kann durch die Anwendung des ersten Teils von dem oben genannten Verfahren nach der vorliegenden Erfindung hergestellt werden bzw. ist derart erhältlich, wobei nur die folgenden Verfahrensschritte durchgeführt werden:
- Erzeugen einer ersten Metallschicht auf einem Substrat,
- Erzeugen einer zweiten Metallschicht über der ersten Metallschicht und
- Herstellen eines Multilayers umfassend eine Anzahl von mehrlagigen Implantatstrukturen über der zweiten Metallschicht.

Da sich die Implantatstrukturen bei diesem Zwischenprodukt noch auf der stabilen Substratschicht befindet, sind die Implantatstrukturen gut geschützt vor mechanischen Belastungen. In diesem Stadium des Zwischenprodukts können die Implantatstrukturen daher auf sichere Weise zur Weiterbearbeitung an einem anderen Ort transportiert werden. Die Zwischenprodukte zur Herstellung von Implantatstrukturen können durch die Anwendung des oben genannten Verfahren nach der vorliegenden Erfindung hergestellt werden, wobei nur die folgenden Verfahrensschritte durchgeführt werden:
- Entfernen der ersten Metallschicht zwischen dem Substrat und der zweiten Metallschicht und
- Ablösen der mehrlagigen Implantatstrukturen in einem zusammenhängenden Verbund von dem Substrat.

Durch die Anwendung des zweiten Teils vom oben genannten Verfahren nach der vorliegenden Erfindung auf das Zwischenprodukt können die Implantatstrukturen von dem Substrat in einem zusammenhängenden Verbund abgelöst werden und liegen somit in der gleichen Form vor, als wenn die Implantatstrukturen nach dem vollständigen erfindungsgemäßen Verfahren mit dem ersten und dem zweiten Verfahrensteil in einem Prozess erzeugt worden wären.

Gemäß einer bevorzugten Ausführungsform ist bei der erfindungsgemäßen Implantatstruktur zwischen der ersten Metallschicht und der zweiten Metallschicht eine dritte Metallschicht auf der ersten Metallschicht vorgesehen. Dabei ist die erste Metallschicht insbesondere durch einen nasschemischen Unterätzungsprozess entfernbar, um als Opferschicht zu fungieren und dadurch den Multilayer von dem Substrat abzulösen.

Weitere Einzelheiten, bevorzugte Ausführungsformen und Vorteile der vorliegenden Erfindung ergeben sich aus der nachfolgenden Beschreibung unter Bezugnahme auf die beigefügten Zeichnungen. Es zeigen:
- Figur 1: eine schematische Darstellung vom Aufbau einer Implantatstruktur beim ersten Schritt einer bevorzugten Ausführungsform des Verfahrens nach der vorliegenden Erfindung;
- Figur 2: eine schematische Darstellung vom Aufbau einer Implantatstruktur beim zweiten Schritt einer bevorzugten Ausführungsform des Verfahrens nach der vorliegenden Erfindung;
- Figur 3: eine schematische Darstellung vom Aufbau einer Implantatstruktur beim dritten Schritt einer bevorzugten Ausführungsform des Verfahrens nach der vorliegenden Erfndung;
- Figur 4: eine schematische Darstellung vom Aufbau einer Implantatstruktur beim vierten Schritt einer bevorzugten Ausführungsform des Verfahrens nach der vorliegenden Erfindung;
- Figur 5: eine schematische Darstellung vom Aufbau einer Implantatstruktur beim fünften Schritt einer bevorzugten Ausführungsform des Verfahrens nach der vorliegenden Erfindung;
- Figur 6: eine schematische Darstellung vom Aufbau einer Implantatstruktur beim sechsten Schritt einer bevorzugten Ausführungsform des Verfahrens nach der vorliegenden Erfindung;
- Figur 7: eine schematische Darstellung vom Aufbau einer Implantatstruktur beim siebten Schritt einer bevorzugten Ausführungsform des Verfahrens nach der vorliegenden Erfindung;
- Figur 8: eine schematische Darstellung vom Aufbau einer Implantatstruktur beim achten Schritt einer bevorzugten Ausführungsform des Verfahrens nach der vorliegenden Erfindung; und
- Figur 9: eine schematische Darstellung von oben auf Implantatstrukturen, die nach dem erfindungsgemäßen Verfahren hergestellt und im Verbund vom Substrat abgelöst wurden.

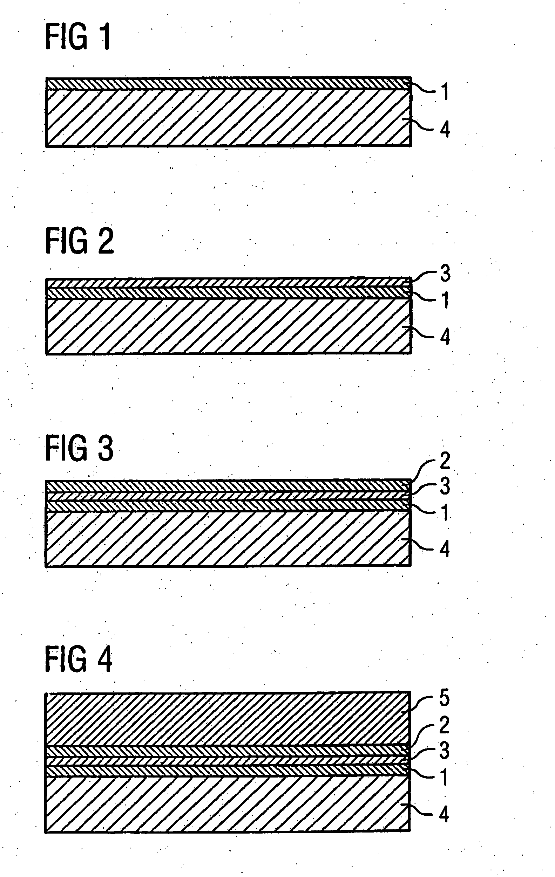

Nachfolgend wird das erfindungsgemäße Verfahren zur Herstellung und Ablösung von Implantatstrukturen im Verbund beschrieben. In Figur 1 ist eine schematische Darstellung vom Aufbau einer Implantatstruktur beim ersten Schritt einer bevorzugten Ausführungsform des Verfahrens nach der vorliegenden Erfindung dargestellt. Das erfindungsgemäße Verfahren baut auf einem Substrat 4 auf, das beispielsweise aus Silizium, Glas oder Keramik besteht. Beim ersten Verfahrensschritt wird zunächst auf dem Substrat 4 eine erste Metallschicht 1 aufgebracht, die zu einem späteren Zeitpunkt des Verfahrens als Ablöseschicht bzw. Opferschicht dient. Diese Opferschicht 1 wird beispielsweise aus Cr oder NiCr hergestellt. Die erste Metallschicht 1 hat vorzugsweise eine Dicke zwischen 70 nm bis 200 nm.

In Figur 2 ist eine schematische Darstellung vom Aufbau einer Implantatstruktur beim zweiten Schritt des erfindungsgemäßen Verfahrens in einer bevorzugten Ausführungsform dargestellt. Bei diesem zweiten Verfahrensschritt wird auf die erste Metallschicht 1 eine dritte Metallschicht 3 aufgetragen. Diese dritte Metallschicht 3 wird beispielsweise aus Titan oder Wolframtitan hergestellt und dient später als Diffusionssperre zwischen der ersten Metallschicht 1 und der zweiten Metallschicht 2. Die dritte Metallschicht 3 hat vorzugsweise eine Dicke von 50 nm bis 200 nm.

In Figur 3 ist eine schematische Darstellung vom Aufbau einer Implantatstruktur beim dritten Schritt des erfiridungsgemäßen Verfahrens in einer bevorzugten Ausführungsform dargestellt. Bei diesem dritten Verfahrensschritt wird auf die dritte Metallschicht 3 eine zweite Metallschicht 2 aufgebracht, so dass die dritte Metallschicht 3 zwischen der ersten Metallschicht 1 und der zweiten Metallschicht 2 liegt. Es hat sich als besonders vorteilhaft erwiesen, wenn die zweite Metallschicht 2 aus Gold hergestellt wird. Die zweite Metallschicht 2 hat vorzugsweise eine Dicke von 50 nm bis 300 nm. Das Erzeugen der Metallschichten 1, 2, 3 kann durch geeignete Verfahren zum Abscheiden dünner Schichten, wie z.B. durch Sputtem oder andere geeignete Verfahren erfolgen.

In Figur 4 ist eine schematische Darstellung vom Aufbau einer Implantatstruktur beim vierten Schritt des erfindungsgemäßen Verfahrens in einer bevorzugten Ausführungsform dargestellt. Bei diesem vierten Verfahrensschritt werden auf der zweiten Metallschicht 2 Implantatstrukturen, wie z.B. flexible Mehrschichtsysteme bzw. Multilayer 5 mit Leiterbahnebenen und Elektroden prozessiert, wobei die flexiblen Schichten des Multilayers 5 beispielsweise aus Polyimid hergestellt werden. Dieser vierte Verfahrensabschnitt des erfindungsgemäßen Verfahrens kann auch mehrere Unterprozesse umfassen, da hierbei die eigentlichen Implantatstrukturen 6 erzeugt werden. Auf diese Weise entsteht über der zweiten Metallschicht 2 ein Multilayer 5 mit mehreren Einzel-Implantatstrukturen 6. Die Prozessierung und Strukturierung des Multilayers 5 erfolgt beispielsweise mit Hilfe von lithographischen Verfahren, die bereits in der Mikrotechnologie etabliert sind.

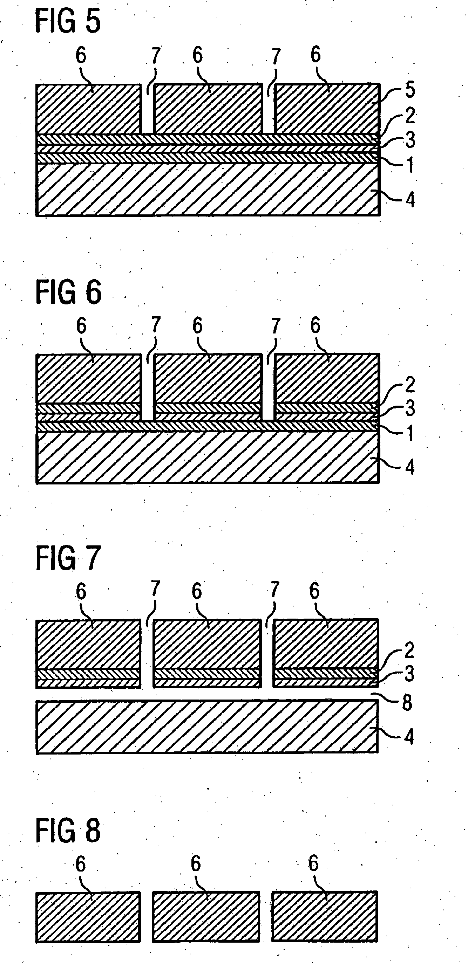

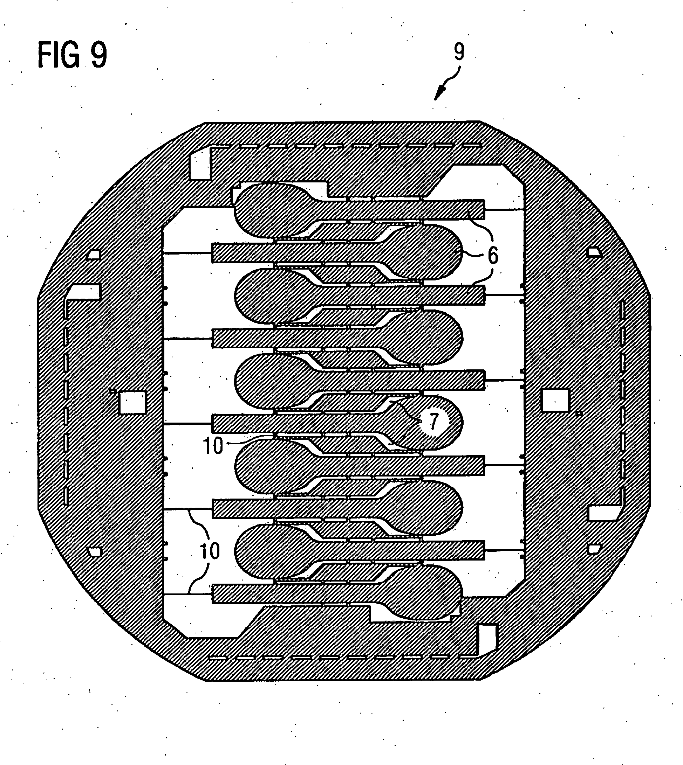

In Figur 5 ist eine schematische Darstellung vom Aufbau einer Implantatstruktur beim fünften Schritt des erfindungsgemäßen Verfahrens in einer bevorzugten Ausführungsform dargestellt. Bei diesem fünften Verfahrensschritt wird die Außengeometrie der Einzel-Implantatstrukturen 6 prozessiert. Durch geeignete Strukturierungsprozesse werden senkrechte Trennspalten 7 zwischen den einzelnen Implantatstrukturen 6 bis auf die zweite Metallschicht 2 erzeugt. Dadurch werden die Implantatstrukturen bzw. Multilayer 6 im Wesentlichen voneinander getrennt, bleiben aber noch über dünne Stege 10 (siehe Fig. 9) miteinander verbunden. Diese Verbindungsstege 10 sind vorzugsweise in der Ebene der Multilayer 6 ausgebildet und genauso aufgebaut wie der Multilayer 5.

In Figur 6 ist eine schematische Darstellung vom Aufbau einer Implantatstruktur beim sechsten Schritt des erfindungsgemäßen Verfahrens in einer bevorzugten Ausführungsform dargestellt. Bei diesem sechsten Verfahrensschritt erfolgt ein nasschemisches Ätzen der beiden oberen Metallschichten 2 und 3, d.h. an den freiliegenden Stellen werden die beiden oberen Metallschichten 2 und 3 entfernt, so dass sich die senkrechten Trennspalten 7 zwischen den Implantatstrukturen 6 bis auf die erste Metallschicht 1 hin erstrecken. Dabei bleibt die unterste Metallschicht bzw. die erste Metallschicht 1 (Opferschicht) noch bestehen.

In Figur 7 ist eine schematische Darstellung vom Aufbau einer Implantatstruktur beim siebten Schritt des erfindungsgemäßen Verfahrens in einer bevorzugten Ausführungsform dargestellt. Bei diesem siebten Verfahrensschritt wird die Opfermetallschicht bzw. erste Metallschicht 1 durch ein nasschemisches Unterätzen vollständig entfernt, wodurch ein waagrechter Trennspalt 8 zwischen dem Substrat 4 und den Implantatstrukturen 6 im Verbund entsteht. Dabei dringt die Ätzlösung von oben durch die Trennspalten 7 ein und beginnt an den freiliegenden Stellen die Implantatstrukturen 6 in lateraler Richtung zu unterätzen, wodurch ein waagrechter Trennspalt 8 erzeugt wird. Aufgrund des waagrechten Trennspalts 8 sind die Implantatstrukturen 6 nun von dem Substrat 4 vollständig und im Verbund abgelöst.

In Figur 8 ist eine schematische Darstellung vom Aufbau einer Implantatstruktur beim achten Schritt des erfindungsgemäßen Verfahrens in einer bevorzugten Ausführungsform dargestellt. Bei diesem achten und letzten Verfahrensschritt werden die beiden auf der Unterseite der Implantatstrukturen 6 verbliebenen Metallschichten 2 und 3 beispielsweise durch geeignete Ätzprozesse entfernt, so dass nur noch die Implantatstrukturen 6 zurückbleiben.

In Figur 9 ist eine schematische Darstellung von oben auf die Implantatstrukturen 6 dargestellt, die nach dem erfindungsgemäßen Verfahren hergestellt und in einem im Verbund vom Substrat abgelöst wurden. Durch die senkrechten Trennspalten 7 sind die Implantatstrukturen 6 im Wesentlichen voneinander getrennt, aber noch über dünne Stege 10 miteinander verbunden. Mit dem erfindungsgemäßen Verfahren können folglich die Implantatsysteme 6 in einem Verbund vom Substrat 4 abgelöst und weiterverarbeitet werden. Dies vereinfacht die Handhabung der dünnen flexiblen Multilayer 6 und erleichtert die nachfolgende Aufbau- und Verbindungstechnik der Implantatsysteme 6 im Hinblick auf die Automatisierung und Prozessierung der Weiterverarbeitung.

### Liste der Bezugszeichen

- 1: erste Metallschicht bzw. Opferschicht
- 2: zweite Metallschicht
- 3: dritte Metallschicht bzw. Diffusionsbarriere
- 4: Substrat
- 5: Multilayer
- 6: Implantatstrukturen
- 7: senkrechte Trennspalten zwischen den Implantatstrukturen 6
- 8: Trennspalt zwischen den Implantatstrukturen 6 und dem Substrat 4
- 9: Verbund von Implantatstrukturen 6
- 10: Stege zwischen den Implantatstrukturen 6

## Patentansprüche

1. Verfahren zum Herstellen von mehrlagigen Implantatstrukturen mit mindestens einer Leiterbahnebene mit elektrischen Leiterbahnen zur elektrischen Kontaktierung von Implantaten, die der Kontaktierung oder Elektrostimulation von lebenden Gewebezellen oder Nerven dienen, umfassend die folgenden Schritte:
• Erzeugen einer ersten Metallschicht (1) auf einem Substrat (4),
• Erzeugen einer zweiten Metallschicht (2) über der ersten Metallschicht (1),
• Herstellen eines Multilayers (5) umfassend eine Anzahl von mehrlagigen Implantatstrukturen (6) über der zweiten Metallschicht (2),
• Entfernen der ersten Metallschicht (1) zwischen dem Substrat (4) und der zweiten Metallschicht (2), und dabei
• Ablösen der mehrlagigen Implantatstrukturen (6) in einem zusammenhängenden Verbund von dem Substrat (4).

2. Verfahren nach Anspruch 1, wobei zwischen dem Erzeugen der ersten Metallschicht (1) und dem Erzeugen der zweiten Metallschicht (2) eine dritte Metallschicht (3) auf der ersten Metallschicht (1) erzeugt wird, so dass die dritte Metallschicht (3) zwischen der ersten Metallschicht (1) und der zweiten Metallschicht (2) ausgebildet wird und als Diffusionssperre zwischen der ersten und zweiten Metallschicht (1, 2) dient.

3. Verfahren nach einem der Ansprüche 1 oder 2, wobei die erste Metallschicht (1) als eine Opferschicht dient, die nach der Herstellung der Implantatstrukturen (6) entfernt wird, um dadurch die erzeugten Implantatstrukturen (6) von dem Substrat (4) zu trennen.

4. Verfahren nach einem der vorangehenden Ansprüche, wobei das Entfernen der ersten Metallschicht (1) zwischen dem Substrat (4) und der zweiten Metallschicht (2) durch einen nasschemischen Unterätzungsprozess erfolgt.

5. Verfahren nach einem der vorangehenden Ansprüche, wobei nach dem Herstellen des Multilayers (5) zwischen den einzelnen Implantatstrukturen (6) mindestens eine Trennspalte (7) erzeugt wird, welche die Implantatstrukturen (6) im Wesentlichen voneinander trennt.

6. Verfahren nach Anspruch 5, wobei sich die Trennspalten (7) zwischen den Implantatstrukturen (6) des Multilayers (5) in senkrechter Richtung bis auf die zweite Metallschicht (2) hin erstrecken.

7. Verfahren nach einem der Ansprüche 5 oder 6, wobei die Implantatstrukturen (6) durch die Trennspalten (7) im Wesentlichen voneinander getrennt werden und über dünne Verbindungsstege (10) miteinander verbunden sind, und wobei die Verbindungsstege (10) vorzugsweise zusammen mit den Implantatstrukturen (6) des Multilayers (5) erzeugt werden.

8. Verfahren nach einem der vorangehenden Ansprüche, wobei um den Multilayer (5) ein starrer Rahmen ausgebildet bzw. angeordnet wird, der die Implantatstrukturen (6) des Multilayers (5) zumindest teilweise umgibt.

9. Verfahren nach einem der Ansprüche 5 bis 8, wobei nach dem Erzeugen der Trennspalten (7) die zweite und dritte Metallschicht (2, 3) an den freiliegenden Stellen durch einen oder mehrere nasschemische Ätzprozesse entfernt werden, so dass sich die senkrechten Trennspalten (7) zwischen den Implantatstrukturen (6) bis zur ersten Metallschicht (1) erstrecken.

10. Verfahren nach Anspruch 9, wobei nach dem Entfernen der zweiten und dritten Metallschicht (2, 3) im Bereich der Trennspalten (7), die erste Metallschicht (1) entfernt wird, wodurch die auf dem Substrat (4) erzeugten Implantatstrukturen (6) von dem Substrat (4) abgelöst werden.

11. Verfahren nach einem der vorangehenden Ansprüche, wobei die erste Metallschicht (1) mit einer Schichtdicke von etwa 70 nm bis 200 nm erzeugt wird.

12. Verfahren nach einem der vorangehenden Ansprüche, wobei die zweite Metallschicht (2) aus einem Material erzeugt wird, das aufgrund seiner Position in der Spannungsreihe der Elemente in Bezug auf das Material der ersten Metallschicht (1) zwischen der ersten und der zweiten Metallschicht (1, 2) eine Potentialdifferenz bewirkt, so dass sich eine elektrochemische Spannung zwischen der ersten Metallschicht (1) und der zweiten Metallschicht (2) ergibt, wobei die zweite Metallschicht (2) bevorzugt aus Gold hergestellt wird.

13. Verfahren nach einem der vorangehenden Ansprüche, wobei die zweite Metallschicht (2) mit einer Schichtdicke von 50 nm bis 300 nm erzeugt wird.

14. Verfahren nach einem der vorangehenden Ansprüche, wobei die dritte Metallschicht (3) mit einer Schichtdicke von 50 nm bis 200 nm erzeugt wird, und bevorzugt aus Titan oder aus Wolframtitan hergestellt wird.

15. Verfahren nach einem der vorangehenden Ansprüche, wobei beim Herstellen der Implantatstrukturen (6) mindestens eine elektrisch isolierende Materialschicht erzeugt wird, auf der eine Leiterbahn-Schicht mit mindestens einer Leiterbahn angeordnet ist.

16. Verfahren nach Anspruch 1, ferner umfassend den folgenden Schritt:
• nach dem Entfernen der ersten Metallschicht (1), Entfernen der zweiten Metallschicht (2) von der Unterseite der zusammenhängenden Implantatstrukturen,
wobei die erste Metallschicht (1) als eine Opferschicht dient,
wobei das Entfernen der ersten Metallschicht (1) zwischen dem Substrat (4) und der zweiten Metallschicht (2) durch einen nasschemischen Unterätzungsprozess erfolgt, und
wobei das Material der zweiten Metallschicht (2) so gewählt ist, dass es aufgrund seiner Position in der Spannungsreihe der Elemente in Bezug auf das Material der ersten Metallschicht (1) eine Potentialdifferenz zu der ersten Metallschicht (1) bewirkt, so dass sich eine elektrochemische Spannung zwischen der ersten Metallschicht (1) und der zweiten Metallschicht (2) ergibt, welche die Auflösung der ersten Metallschicht (1) beim Unterätzungsprozess und damit die Ablösung der Implantatstrukturen von dem Substrat (4) begünstigt.

17. Zwischenprodukt zur Herstellung einer Implantatstruktur (6) mindestens umfassend die folgenden Schichten:
• ein Substrat (4),
• eine erste Metallschicht (1) auf dem Substrat (4),
• eine zweite Metallschicht (2) über der ersten Metallschicht (1), und
• mindestens einen Multilayer (5) über der zweiten Metallschicht (2), wobei der Multilayer (5) eine Anzahl von zusammenhängenden mehrlagigen Implantatstrukturen mit elektrischen Leiterbahnen zur elektrischen Kontaktierung von Implantaten, die der Kontaktierung oder Elektrostimulation von lebenden Gewebezellen oder Nerven dienen, umfasst.

18. Zwischenprodukt zur Herstellung einer Implantatstruktur (6) nach Anspruch 17, das durch die folgenden Schritte hergestellt wurde bzw. erhältlich ist:
• Erzeugen einer ersten Metallschicht auf einem Substrat,
• Erzeugen einer zweiten Metallschicht über der ersten Metallschicht und
• Herstellen eines Multilayers umfassend eine Anzahl von mehrlagigen Implantatstrukturen über der zweiten Metallschicht.

19. Zwischenprodukt zur Herstellung einer Implantatstruktur (6) nach einen der Ansprüche 17 oder 18, wobei zwischen der ersten Metallschicht (1) und der zweiten Metallschicht (2) eine dritte Metallschicht (3) auf der ersten Metallschicht (1) vorgesehen ist.

## Claims

1. Method for producing multilayered implant structures with at least one conductor-track plane having electrical conductor tracks for the electrical contacting of implants that serve for contacting or electrostimulation of living tissue cells or nerves, comprising the following steps:
• generating a first metal layer (1) on a substrate (4),
• generating a second metal layer (2) above the first metal layer (1),
• producing a multilayer (5) comprising a number of multilayered implant structures (6) above the second metal layer (2),
• removing the first metal layer (1) between the substrate (4) and the second metal layer (2), and thereby
• releasing the multilayered implant structures (6) from the substrate (4) in a coherent composite.

2. Method according to Claim 1, wherein, between the generating of the first metal layer (1) and the generating of the second metal layer (2), a third metal layer (3) is generated on the first metal layer (1), so that the third metal layer (3) is formed between the first metal layer (1) and the second metal layer (2) and serves as diffusion barrier between the first and second metal layers (1, 2).

3. Method according to one of Claims 1 or 2, wherein the first metal layer (1) serves as a sacrificial layer which is removed after the production of the implant structures (6), thereby to separate the produced implant structures (6) from the substrate (4).

4. Method according to one of the preceding claims, wherein the removal of the first metal layer (1) between the substrate (4) and the second metal layer (2) is effected by a wet-chemical under-etching process.

5. Method according to one of the preceding claims, wherein, after the production of the multilayer (5) between the individual implant structures (6), at least one separating gap (7) is generated which substantially separates the implant structures (6) from one another.

6. Method according to Claim 5, wherein the separating gaps (7) between the implant structures (6) of the multilayer (5) extend in the vertical direction as far as the second metal layer (2).

7. Method according to one of Claims 5 or 6, wherein the implant structures (6) are substantially separated from one another by the separating gaps (7) and are connected to one another via thin connecting webs (10), and wherein the connecting webs (10) preferably are generated together with the implant structures (6) of the multilayer (5).

8. Method according to one of the preceding claims, wherein a rigid frame which at least partially surrounds the implant structures (6) of the multilayer (5) is formed or arranged around the multilayer (5).

9. Method according to one of Claims 5 to 8, wherein, after the generation of the separating gaps (7), the second and third metal layers (2, 3) are removed at the exposed sites by one or more wet-chemical etching processes, so that the vertical separating gaps (7) extend between the implant structures (6) as far as the first metal layer (1).

10. Method according to Claim 9, wherein, after the removal of the second and third metal layers (2, 3) in the region of the separating gaps (7), the first metal layer (1) is removed, whereby the implant structures (6) generated on the substrate (4) are detached from the substrate (4).

11. Method according to one of the preceding claims, wherein the first metal layer (1) is generated with a layer thickness from about 70 nm to 200 nm.

12. Method according to one of the preceding claims, wherein the second metal layer (2) is generated from a material that, by reason of its position in the electrochemical series of the elements in relation to the material of the first metal layer (1), brings about a potential difference between the first and the second metal layers (1, 2), so that an electrochemical voltage arises between the first metal layer (1) and the second metal layer (2), wherein the second metal layer (2) preferably is produced from gold.

13. Method according to one of the preceding claims, wherein the second metal layer (2) is generated with a layer thickness from 50 nm to 300 nm.

14. Method according to one of the preceding claims, wherein the third metal layer (3) is generated with a layer thickness from 50 nm to 200 nm, and preferably is produced from titanium or from tungsten titanium.

15. Method according to one of the preceding claims, wherein in the course of producing the implant structures (6) at least one electrically insulating material layer is generated, on which a conductor-track layer with at least one conductor track is arranged.

16. Method according to Claim 1, further comprising the following step:
• after removing the first metal layer (1), removing the second metal layer (2) from the bottom side of the coherent implant structures,
wherein the first metal layer (1) serves as a sacrificial layer,
wherein the removal of the first metal layer (1) between the substrate (4) and the second metal layer (2) is effected by a wet-chemical under-etching process, and
wherein the material of the second metal layer (2) is selected so that, by reason of its position in the electrochemical series of the elements in relation to the material of the first metal layer (1), it brings about a potential difference towards the first metal layer (1), so that an electrochemical voltage arises between the first metal layer (1) and the second metal layer (2), which promotes the dissolution of the first metal layer (1) during the under-etching process and thereby the detachment of the implant structures from the substrate (4).

17. Intermediate product for producing an implant structure (6) at least comprising the following layers:
• a substrate (4),
• a first metal layer (1) on the substrate (4),
• a second metal layer (2) above the first metal layer (1), and
• at least one multilayer (5) above the second metal layer (2), wherein the multilayer (5) comprises a number of coherent multilayered implant structures with electrical conductor tracks for the electrical contacting of implants that serve for contacting or electrostimulation of living tissue cells or nerves.

18. Intermediate product for producing an implant structure (6) according to Claim 17 that has been produced or is obtainable by means of the following steps:
• generating a first metal layer on a substrate,
• generating a second metal layer above the first metal layer, and
• producing a multilayer comprising a number of multilayered implant structures above the second metal layer.

19. Intermediate product for producing an implant structure (6) according to one of Claims 17 or 18, wherein between the first metal layer (1) and the second metal layer (2) a third metal layer (3) is provided on the first metal layer (1).

## Revendications

1. Procédé de fabrication de structures d'implant à couches multiples avec au moins un niveau de piste conductrice comprenant des pistes conductrices électriques pour la mise en contact électrique d'implants qui servent à la mise en contact ou à l'électrostimulation de cellules tissulaires vivantes ou de nerfs, comprenant les étapes suivantes :
- production d'une première couche métallique (1) sur un substrat (4),
- production d'une deuxième couche métallique (2) sur la première couche métallique (1),
- fabrication d'un circuit multicouche (5) comprenant une pluralité de structures d'implant à couches multiples (6) sur la deuxième couche métallique (2),
- enlèvement de la première couche métallique (1) entre le substrat (4) et la deuxième couche métallique (2), et ce faisant
- dépouillement des structures d'implant à couches multiples (6) du substrat (4) dans une interconnexion d'un seul tenant.

2. Procédé selon la revendication 1, dans lequel entre la production de la première couche métallique (1) et la production de la deuxième couche métallique (2), une troisième couche métallique (3) est produite sur la première couche métallique (1), de telle sorte que la troisième couche métallique (3) est formée entre la première couche métallique (1) et la deuxième couche métallique (2) et sert de barrière de diffusion entre les première et deuxième couches métalliques (1, 2).

3. Procédé selon la revendication 1 ou 2, dans lequel la première couche métallique (1) sert de couche sacrificielle qui est enlevée après la fabrication des structures d'implant (6) afin de séparer ainsi les structures d'implant (6) produites du substrat (4).

4. Procédé selon l'une des revendications précédentes, dans lequel l'enlèvement de la première couche métallique (1) entre le substrat (4) et la deuxième couche métallique (2) est réalisé par un processus de gravure sous-jacente par voie humide.

5. Procédé selon l'une des revendications précédentes, dans lequel après la fabrication du circuit multicouche (5) entre les structures d'implant (6) individuelles, au moins une fente de séparation (7) est produite, laquelle sépare essentiellement les structures d'implant (6) les unes des autres.

6. Procédé selon la revendication 5, dans lequel les fentes de séparation (7) s'étendent en direction perpendiculaire entre les structures d'implant (6) du circuit multicouche (5) jusqu'à la deuxième couche métallique (2).

7. Procédé selon l'une des revendications 5 ou 6, dans lequel les structures d'implant (6) sont essentiellement séparées les unes des autres par les fentes de séparation (7) et sont reliées les unes avec les autres au moyen de minces barrettes de liaison (10), étant entendu que les barrettes de liaison (10) sont de préférence produites en conjonction avec les structures d'implant (6) du circuit multicouche (5).

8. Procédé selon l'une des revendications précédentes, dans lequel un cadre rigide est réalisé ou agencé autour du circuit multicouche (5), lequel cadre entoure au moins partiellement les structures d'implant (6) du circuit multicouche (5).

9. Procédé selon l'une des revendications 5 à 8, dans lequel après la production des fentes de séparation (7), les deuxième et troisième couches métalliques (2, 3) sont enlevées dans les zones découvertes par un ou plusieurs processus de gravure par voie humide, de telle sorte que les fentes de séparation (7) perpendiculaires s'étendent entre les structures d'implant (6) jusqu'à la première couche métallique (1).

10. Procédé selon la revendication 9, dans lequel après l'enlèvement des deuxième et troisième couches métalliques (2, 3) dans la zone des fentes de séparation (7), la première couche métallique (1) est enlevée, ce par quoi les structures d'implant (6) produites sur le substrat (4) sont dépouillées du substrat (4).

11. Procédé selon l'une des revendications précédentes, dans lequel la première couche métallique (1) est produite avec une épaisseur de couche d'environ 70 nm à 200 nm.

12. Procédé selon l'une des revendications précédentes, dans lequel la deuxième couche métallique (2) est produite à partir d'un matériau qui, en raison de sa position dans la série électrochimique des éléments par rapport au matériau de la première couche métallique (1), produit une différence de potentiel entre les première et deuxième couches métalliques (1, 2), de telle sorte qu'une force électrochimique est créée entre la première couche métallique (1) et la deuxième couche métallique (2), étant entendu que la deuxième couche métallique (2) est de préférence fabriquée à partir d'or.

13. Procédé selon l'une des revendications précédentes, dans lequel la deuxième couche métallique (2) est produite avec une épaisseur de couche de 50 nm à 300 nm.

14. Procédé selon l'une des revendications précédentes, dans lequel la troisième couche métallique (3) est produite avec une épaisseur de couche de 50 nm à 200 nm et elle est de préférence fabriquée à partir de titane ou de titane-tungstène.

15. Procédé selon l'une des revendications précédentes, dans lequel lors de la fabrication des structures d'implant (6), au moins une couche de matériau isolante électriquement est produite, sur laquelle est agencée une couche de piste conductrice comprenant au moins une piste conductrice.

16. Procédé selon la revendication 1, comprenant en outre l'étape suivante :
- après l'enlèvement de la première couche métallique (1), enlèvement de la deuxième couche métallique (2) du côté inférieur des structures d'implant d'un seul tenant,
étant entendu que la première couche métallique (1) sert de couche sacrificielle,
étant entendu que l'enlèvement de la première couche métallique (1) entre le substrat (4) et la deuxième couche métallique (2) est réalisé par un processus de gravure sous-jacente par voie humide, et
étant entendu que le matériau de la deuxième couche métallique (2) est choisi de telle sorte qu'en raison de sa position dans la série électrochimique des éléments par rapport au matériau de la première couche métallique (1), il produit une différence de potentiel avec la première couche métallique (1), de telle sorte qu'une force électrochimique est créée entre la première couche métallique (1) et la deuxième couche métallique (2), laquelle favorise la dissolution de la première couche métallique (1) lors du processus de gravure sous-jacente et, partant, le dépouillement des structures d'implant du substrat (4).

17. Produit intermédiaire destiné à la fabrication d'une structure d'implant (6) comprenant au moins les couches suivantes :
- un substrat (4),
- une première couche métallique (1) sur le substrat (4),
- une deuxième couche métallique (2) sur la première couche métallique (1), et
- au moins un circuit multicouche (5) sur la deuxième couche métallique (2), étant entendu que le circuit multicouche (5) comprend une pluralité de structures d'implant à couches multiples d'un seul tenant avec des pistes conductrices électriques pour la mise en contact électrique d'implants, qui servent à la mise en contact ou à l'électrostimulation de cellules tissulaires vivantes ou de nerfs.

18. Produit intermédiaire destiné à la fabrication d'une structure d'implant (6) selon la revendication 17 qui a été fabriqué ou qui peut être obtenu par les étapes suivantes :
- production d'une première couche métallique sur un substrat,
- production d'une deuxième couche métallique sur la première couche métallique, et
- fabrication d'un circuit multicouche comprenant une pluralité de structures d'implant à couches multiples sur la deuxième couche métallique.

19. Produit intermédiaire destiné à la fabrication d'une structure d'implant (6) selon l'une des revendications 17 ou 18, étant entendu qu'entre la première couche métallique (1) et la deuxième couche métallique (2), il est prévu une troisième couche métallique (3) sur la première couche métallique (1).
